# DEMANDE DE BREVET EUROPEEN

(11) **EP 1 066 795 A1**
(43) Date de publication de la demande: **10.01.2001**
(21) Numéro de dépôt: 00401966.7
(22) Date de dépôt: 07.07.2000
(51) Int. Cl.: A61B 10/00

(54) **Pince à biopsie endoscopique**

(30) Priorité: 09.07.1999 FR 9908936
(71) Demandeur: Odon Life Technology, 92200 Neuilly sur Seine (FR)
(72) Inventeur: Oblin, Jacques, 14123 Cormelles le Royal (FR); Durand, Jean-Paul, 92380 Garches (FR)
(74) Mandataire: Bloch, Gérard

(57) **Abrégé**

La pince comprend une gaine externe (10), des moyens de coupe (3,4), à une extrémité distale de la gaine, montés sur une extrémité distale d'un câble (5) s'étendant à l'intérieur de la gaine (10), des moyens (2) d'actionnement des moyens de coupe (3,4), aux extrémités proximales de la gaine et du câble, une gaine interne (8), mobile dans la gaine externe (10) et dans laquelle s'étend le câble (5), la gaine externe (10) et le câble (5) étant fixes en déplacement et les moyens d'actionnement (2) étant agencés pour entraîner la gaine interne (8) en déplacement dans la gaine externe (10).

## Description

Une pince à biopsie endoscopique est une pince qu'on introduit dans un canal opérateur d'un endoscope, dans lequel on la fait avancer jusqu'à ce qu'elle apparaisse dans le champ de vision, à proximité d'une zone dont il faut couper et prélever des tissus.

On connaît déjà un premier type de pince à biopsie endoscopique, dit à pantographe, comprenant une gaine, destinée à être immobilisée dans le canal opérateur de l'endoscope, une paire de cuillers montées pivotantes sur l'une (distale) des extrémités de la gaine, un cable, de traction et d'entraînement des cuillers en pivotement, monté mobile dans la gaine, et, à l'autre extrémité proximale de la gaine, une poignée de manoeuvre et de mise en traction du câble pour provoquer son déplacement dans la gaine et donc le pivotement des cuillers, le pincement des tissus puis leur coupe et leur prélèvement.

Généralement, la gaine est solidaire de la poignée et d'un anneau de réception du pouce d'une main du praticien et le câble de traction est solidaire d'une tirette montée coulissante sur la poignée, le pincement des tissus étant provoqué par action sur les doigts tendant à les regrouper.

La pince à pantographe est satisfaisante et parfaitement éprouvée mais le montage des cuillers sur un axe solidaire de la gaine implique des contraintes de précisions proches de celles de l'industrie horlogère qui sont par exemple peu compatibles avec une production de masse de pinces à usage unique.

On connaît aussi un autre type de pince à biopsie endoscopique, voisin du premier type, dans lequel les cuillers ne sont pas montées pivotantes sur la gaine, mais prolongent le câble tracteur. Si une telle pince est d'un coût satisfaisant, son emploi soulève des difficultés. Les cuillers sont solidaires en déplacement du câble ; quand on tire sur le câble, on rapproche les cuillers, quand on repousse le câble dans le fourreau, on les écarte. Pour rapprocher les cuillers, il faut donc que le praticien exerce sur ses doigts une action contraire à celle qu'il doit exercer avec une pince à pantographe. De surcroît, et surtout, ayant fait avancer la pince jusqu'à la paroi à biopsier, le rapprochement des cuillers, provoqué par une traction sur le câble, s'accompagne donc d'une rétractation de celles-ci dans la gaine sur une distance non négligeable, ce qui affecte la précision.

La présente invention vise à pallier au moins l'un ou l'autre de ces inconvénients.

A cet effet, l'invention concerne une pince à biopsie endoscopique comprenant
- une gaine externe,
- des moyens de coupe, à une extrémité distale de la gaine, montés sur une extrémité distale d'un câble s'étendant à l'intérieur de la gaine,
- des moyens d'actionnement des moyens de coupe, aux extrémités proximales de la gaine et du câble,
pince caractérisée par le fait qu'elle comprend, en outre,
- une gaine interne, montée mobile dans la gaine externe et dans laquelle s'étend le câble des moyens de coupe,
- la gaine externe et le câble étant fixes en déplacement lors de l'actionnement des moyens de coupe et
- les moyens d'actionnement des moyens de coupe étant agencés pour entraîner la gaine interne en déplacement dans la gaine externe.

Les moyens de coupe étant immobilisés en déplacement dans l'endoscope, avec la gaine externe, contre la paroi à biopsier, leur actionnement n'est dû qu'au déplacement relatif de la gaine interne, sans qu'ils ne reculent et s'écartent donc de la paroi.

De préférence, les moyens d'actionnement des moyens de coupe sont agencés pour provoquer l'actionnement par rapprochement des extrémités proximales des deux gaines.

Ainsi, c'est par une action classique sur les doigts de sa main que le praticien peut procéder à la biopsie.

Avantageusement, les moyens de coupe comprennent une paire d'organes de pincement agencés pour être rapprochés l'un de l'autre par l'extrémité distale de la gaine interne.

L'invention sera mieux comprise à l'aide de la description suivante d'une forme de réalisation préférée de la pince de l'invention, en référence au dessin annexé, sur lequel
- la figure 1 est une vue en perspective éclatée de la pince;
- la figure 2 est une vue en coupe de l'extrémité distale opérationnelle de la pince et
- les figures 3A et 3B illustrent le fonctionnement de la pince.

La pince comporte, à une extrémité distale, la pince 1 proprement dite, c'est-à-dire l'instrument de pincement, de coupe et de prélèvement, et, à l'autre extrémité, qui est l'extrémité proximale, une poignée de manoeuvre 2 d'actionnement de l'instrument 1.

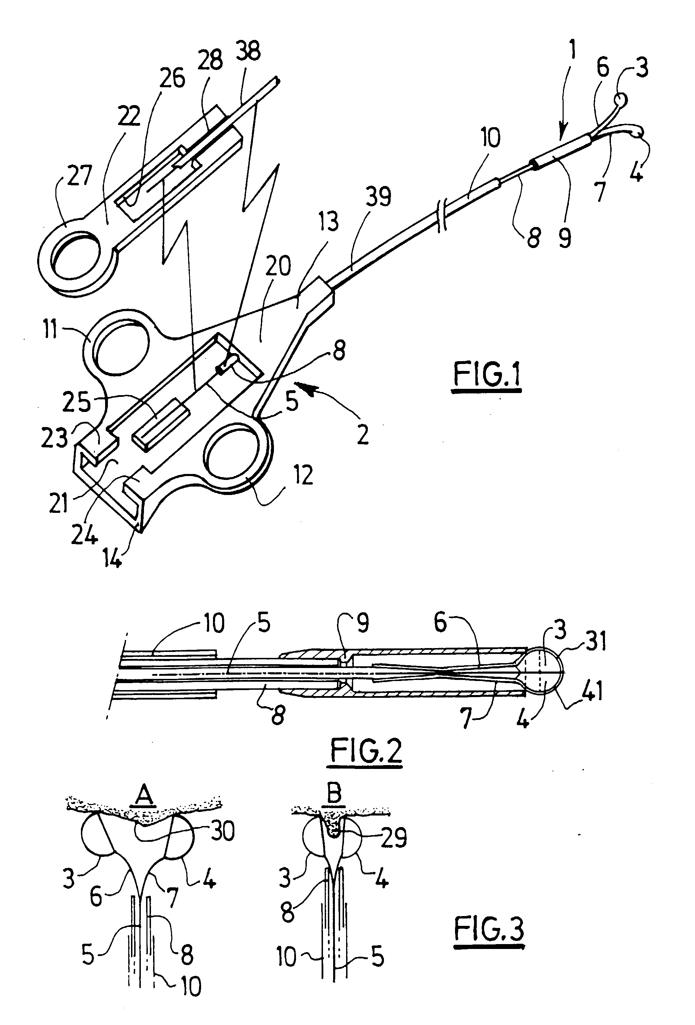

En référence à la figure 2, l'instrument comporte deux petites lames hémisphériques 3, 4 à bords coupants 31, 41, en forme de cuiller ou de coupelle, rapportées sur l'extrémité distale d'un câble 5 en position écartées l'une de l'autre (figure 1 et 3A) pour que l'ensemble soit considéré à mémoire de forme et qu'après avoir forcé ces cuillers l'une contre l'autre (figures 2 et 3B) et les avoir ensuite relachées, elles reprennent leur position écartée l'une de l'autre (figure 1 et 3A).

On notera qu'ici les cuillers 3, 4 prolongent des bras 6, 7 légèrement en V, ici soudés sur le câble 5, pour assurer une bonne ouverture ainsi qu'une bonne fermeture de la pince.

Pour effectuer la fermeture de l'instrument de pince 1, on a placé le câble 5 dans une première gaine 8, qui est une gaine interne, ici prolongée par un étui d'extrémité distale 9, ici soudé sur la gaine 8, et qui peut être entraîné en déplacement autour du câble 5 pour que l'étui 9 recouvre les bras 6, 7 des cuillers en les rapprochant l'une vers l'autre jusqu'à ce que les cuillers 3, 4 viennent en contact l'une contre l'autre en formant pratiquement une sphère (figure 2).

On notera que l'étui 9, qui n'est pas strictement indispensable, vise essentiellement à assurer la force nécessaire à la fermeture de la pince, en tout cas, à assurer cette force mieux que ne le ferait la gaine 8 seule.

Pour pouvoir entraîner la gaine interne 8 en déplacement le long ou autour du câble 5, on a placé la gaine interne 8 dans une deuxième gaine 10, qui est une gaine externe destinée à être immobilisée dans un canal opérateur d'endoscope, et on a solidarisé en déplacement la gaine externe 10 et le câble 5 des cuillers de coupe 3, 4, la gaine interne 8, dans lequel s'étend le câble 5, étant montée mobile dans la gaine externe 10.

Pour actionner la pînce 1 et provoquer le déplacement relatif de la gaine interne 8, ici avec son étui 9, et de la gaine externe 10 et du câble 5, on les a tous les trois fixés à des éléments de la poignée de manoeuvre 2 qui va maintenant être décrite.

Il s'agit globalement d'une plaque 20, avec deux anneaux de préhension 11, 12, dans lesquels le praticien doit introduire l'index et le majeur de sa main de manoeuvre, et à une extrémité 13 de laquelle, ici rétrécie et opposée à l'extrémité proximale 14 de la plaque 20, est fixé la gaine externe 10.

Il est ménagée dans la plaque 20 une plage 21 de réception d'une tirette 22 dans laquelle est ancrée l'extrémité proximale de la gaine interne 8. A l'extrémité proximale de la plaque 20, deux rebords 23, 24 surplombent la plage 21 pour former une coulisse pour la tirette 22.

Un plot 25 d'ancrage de l'extrémité proximale du câble 5 est disposé sur la plage 21. La tirette 22, qu'on voit retournée sur la figure 1, comporte un évidement 26, formant lumière de réception pour le plot 25, pour permettre son coulissement sur la plage 21 de la plaque 20 sans qu'il ne soit entravé par le plot d'ancrage 25.

L'extrémité proximale de la tirette 22 est conformée en anneau de préhension 27 dans lequel le praticien doit introduire son pouce. Il est ménagé à l'extrémité distale de la tirette 22 une fente 28 d'ancrage de l'extrémité proximale de la gaine interne 8.

Ainsi, et en partant du bord proximal de la plaque de manoeuvre 20, on trouve l'extrémité proximale du câble 5 des cuillers de coupe 3, 4, ancrée dans le plot 25, l'extrémité proximale de la gaine interne 8, ancrée dans la tirette 22, et l'extrémité proximale de la gaine externe 10, le câble 5 et le fourreau externe 10 étant solidaires entre eux par l'intermédiaire de la plaque 20 et la gaine interne 8 étant solidaire de la tirette 22.

Le fonctionnement de la pince à biopsie endoscopique va maintenant être décrit.

On fait avancer l'instrument de pince 1 dans le canal opérateur d'un endoscope jusqu'à ce que les cuillers de coupe 3, 4, en position d'ouverture de la pince, viennent en contact avec la zone 30 dont il faut couper et prélever des tissus (figure 3A). La pince est ouverte, c'est-à-dire que l'extrémité distale de la gaine interne 8 (l'étui 9) est en position reculée sur le câble 5. Par rapprochement de ses trois doigts glissés dans les anneaux 11, 12, 27 de la poignée de manoeuvre 2, le praticien repousse l'extrémité distale de la gaine interne 8 vers les cuillers pour qu'elle vienne recouvrir les bras 6, 7, rapprocher les cuillers 3, 4 l'une de l'autre pour qu'elles pincent une partie 29 des tissus et la coupe pour la détacher de la zone 30, grâce à leurs bords coupants 31, 41, l'extrémité distale de la gaine 8 étant alors en butée contre les cuillers.

On remarquera que lors de l'actionnement classique de la poignée de manoeuvre 2 par le praticien et du déplacement relatif des anneaux 11, 12, 27, les extrémités proximales 38, 39, des deux gaines 8, 10, solidaires respectivement de la tirette 22 et de la poignée 2, sont rapprochées l'une de l'autre.

A titre purement informatif et d'exemple, les cuillers de coupe 3, 4 peuvent être en acier inox et obtenues par emboutissage, le câble 5 de ces cuillers être également en acier inox, de même que la gaine intérieure et son étui, la gaine extérieure être en polyamide, la poignée de manoeuvre être en polyéthylène haute densité. La gaine intérieure pourra être en acier spiralé.

On peut vouloir protéger la pince qui vient d'être décrite de toute contamination lors de son passage dans un canal opérateur d'endoscope; la protection doit surtout concerner la partie distale et active de la pince qui est initialement stérile pour que le patient, lors de la biopsie, ne soit pas exposé à un risque infectieux.

On rappellera, en référence à la pince des figures 1 à 3, que la gaine externe 10 et le câble 5 ont été solidarisés en déplacement, la gaine 10 ayant été fixée à une extrémité rétrécie 13 de la plaque 20 de la poignée de manoeuvre 2.

Et bien, aux fins de protection de la partie distale de la pince, on peut prévoir facilement que la gaine externe 10 puisse occuper deux états, un premier état déployé et un deuxième état reployé. Il s'agirait d'une gaine externe à deux fonctions. Dans son état déployé, la gaine 10 pourrait recouvrir les cuillers de coupe 3, 4, un bouchon, par exemple de paraffine médicale, obturant son extrémité pour finir de protéger les cuillers de coupe. Dans son état reployé, la gaine 10 assurerait sa fonction de coupe. C'est le passage de l'état déployé à l'état reployé qui provoquerait l'expulsion du bouchon de protection par les cuillers de coupe.

## Revendications

1. Pince à biopsie endoscopique comprenant
- une gaine externe (10),
- des moyens de coupe (3, 4), à une extrémité distale de la gaine, montés sur une extrémité distale d'un câble (5) s'étendant à l'intérieur de la gaine (10),
- des moyens (2) d'actionnement des moyens de coupe (3, 4), aux extrémités proximales de la gaine et du câble,
pince caractérisée par le fait qu'elle comprend, en outre,
- une gaine interne (8), montée mobile dans la gaine externe (10) et dans laquelle s'étend le câble (5) des moyens de coupe (3, 4),
- la gaine externe (10) et le câble (5) étant fixes en déplacement lors de l'actionnement des moyens de coupe et
- les moyens (2) d'actionnement des moyens de coupe (3, 4) étant agencés pour entraîner la gaine interne (8) en déplacement dans la gaine externe (10).

2. Pince selon la revendication 1, dans laquelle les moyens (2) d'actionnement des moyens de coupe (3, 4) sont agencés pour provoquer l'actionnement par rapprochement des extrémités proximales (38, 39) des deux gaines (8, 10).

3. Pince selon l'une des revendications 1 et 2, dans laquelle les moyens de coupe comprennent une paire d'organes de pincement (3, 4) agencés pour être rapprochés l'un de l'autre par l'extrémité distale (9) de la gaine interne (8).

4. Pince selon l'une des revendications 1 à 3, dans laquelle lesdits moyens d'actionnement comprennent une poignée de manoeuvre (2), de laquelle la gaine externe (10) et le câble (5) des moyens de coupe (3, 4) sont solidaires en actionnement.

5. Pince selon la revendication 4, dans laquelle une tirette (22), de laquelle la gaine interne (8) est solidaire, est montée coulissante sur la poignée (2).

6. Pince selon l'une des revendications 1 à 5, dans laquelle la gaine interne (8) est prolongée par un étui d'extrémité distale (9).

7. Pince selon l'une des revendications 1 à 6, dans laquelle la gaine externe (10) est agencée pour pouvoir recouvrir les moyens de coupe (3, 4).
